# EUROPEAN PATENT SPECIFICATION

(11) **EP 2 090 261 B1**
(45) Date of publication and mention of the grant of the patent: **01.06.2011**
(21) Application number: 08396004.7
(22) Date of filing: 18.02.2008
(51) Int. Cl.: A61B 19/02

(54) **Movable equipment for medical environment**
Bewegliche Ausrüstung für eine medizinische Umgebung
Équipement mobile pour environnement médical

(43) Date of publication of application: 19.08.2009
(73) Proprietor: GENERAL ELECTRIC COMPANY, Schenectady, NY 12345 (US)
(72) Inventor: Vilkas, Vesa, 02760 Espoo (FI)
(74) Representative: Kanerva, Arto

(56) References cited:
- WO-A-2007/031759
- DE-U- 7 532 292
- DE-U1- 8 434 471
- US-A- 5 518 310
- US-A1- 2007 176 060

## Description

### BACKGROUND OF THE INVENTION

This disclosure relates generally to a movable equipment for a medical environment.

Hospital efficiency requirements get more demanding, more patients have to be taken through surgery process in shorter time. A personnel is busy and this causes potential risky situations while moving machines and they cannot spend much time in front of machines to constantly watch machine status. Anesthesia machines are used for anesthetizing and ventilating patients in hospital operating rooms, intensive care units or other patient care places. Usually the anesthesia machine is mounted in a cart with wheels and can be moved around easily. Other ways of mounting it are on a wall or in a ceiling or a mobile version that could be mounted in many locations.

Operating rooms are quite small and full of different machines. There is not much space for a hospital personnel or for equipment. The operating room floor is full of different cables e.g. for measuring a heart rate, a blood pressure and other parameters, but as well for a power and a signal etc. Also there are a lot of tubes e.g. for breathing, intravenous fluids etc. between the anesthesia machine and the patient. When the anesthesia machine is moved it can easily run over cables and tubes causing a damage. This can happen either during an operation or during a transportation when the anesthesia machine is moved to another location. Some operations are executed in dimmed conditions and then it is even harder to see cables and tubes on the floor. During the transportation there are doorsills and other obstacles, probably in a poorly illuminated environment causing dangerous situations.

Utility model DE 7532292 discloses a device for a medical revival and intensive care having a tiltable lamp fixed to a horizontal holder of the device.
US-A-5 518 310 refers to a mobile cast having lamps for illuminating a surgical site.

Nowadays there is no integrated solution in the machine that helps to see cables and tubes under the machine or that helps to see obstacles during the transportation.

Further an operational status of the anesthesia machine cannot be seen easily from far, a user does not see whether the anesthesia machine is operating normally or having some problems. These situations include abnormal conditions like a hospital main power failure, some failure in a normal operation or other special situation that requires the user's attention. The user can see a status of the machine from a user interface monitor but this requires going close to the machine. For example the user would want to see clearly when the machine is ready for a first operation in a morning and the machine has performed automated tests.

Also a condition of the patient is not well visible from far away. The anesthesia machine has audible alarms for emergency cases but before that it would be helpful to see what will likely happen soon through some indication. Especially three states of a surgery are not clearly visible: a beginning of the surgery when the patient is put to sleep, a steady state when the patient is in sleep and an end of the surgery when the patient is waking up could not be identified rapidly in cases when someone enters the operating room suddenly.

Further the operating room floor gets dirty during the operation and can have a lot of bacteria. A blood and other excretions cause contaminations and this causes a potential risk of diseases. The floor is cleaned after the operation but it may still be disinfected.

### BRIEF DESCRIPTION OF THE INVENTION

The above-mentioned shortcomings, disadvantages and problems are addressed herein which will be understood by reading and understanding the following specification.
The invention is defined by claim 1.

In an embodiment a movable equipment for a medical environment includes a frame having a place for a medical apparatus and a moving member for moving the frame. The movable equipment also includes at least one light source for illuminating at least a part of a space adjacent to the frame. The at least one light source is configured to illuminate a floor around the frame or a floor below the frame.

In another embodiment, a movable equipment for a medical environment includes a frame, a place in the frame for a medical apparatus and a moving member coupled to the frame. The movable equipment also includes at least one light source for illuminating a floor adjacent to the frame. The at least one light source is configured to illuminate a floor around the frame or a floor below the frame.

In yet another embodiment a movable equipment for a medical environment includes a frame having a place for an anesthesia machine and a moving member for the frame. The movable equipment further comprises at least one light source for illuminating a floor adjacent to the frame. The at least one light source is configured to illuminate a floor around the frame or a floor below the frame.

Various other features, objects, and advantages of the invention will be made apparent to those skilled in art from the accompanying drawings and detailed description thereof.

### BRIEF DESCRIPTION OF THE DRAWINGS

Figure 1 is a general view of an apparatus of an embodiment; and

Figure 2 is a general view of an apparatus of another embodiment.

### DETAILED DESCRIPTION OF THE INVENTION

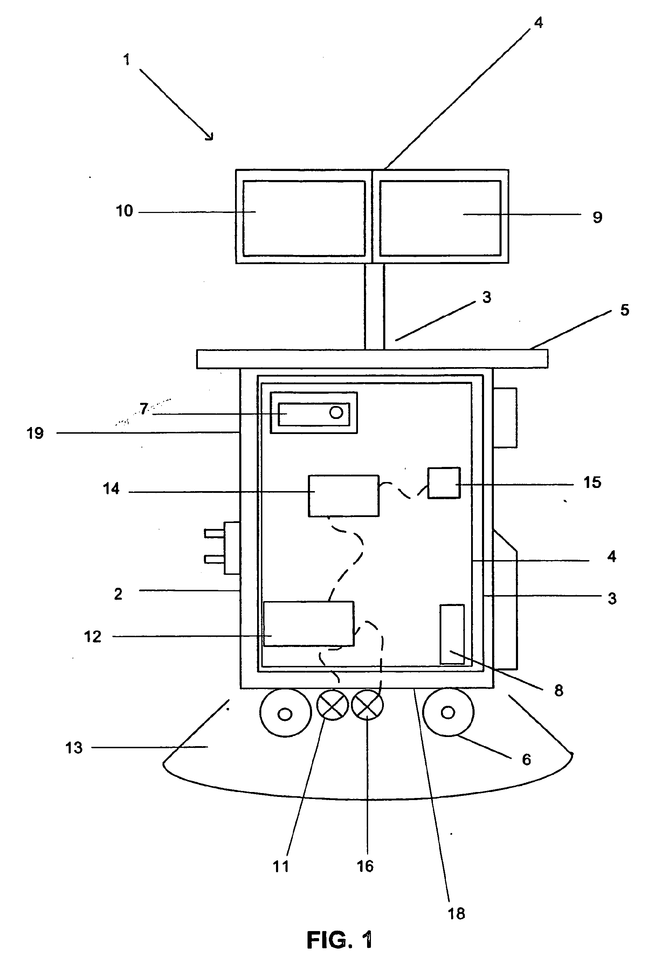

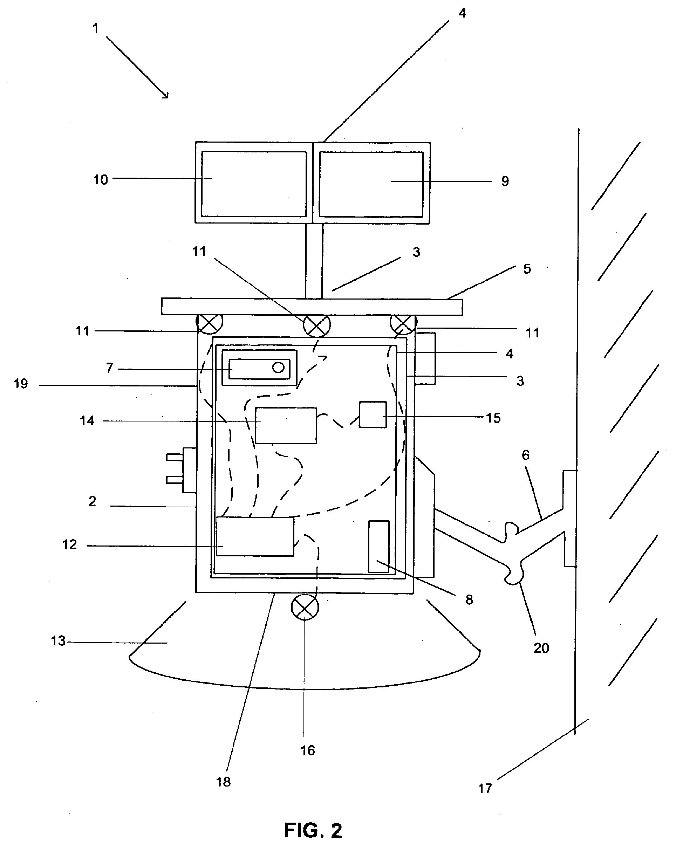

Figure 1 shows a movable equipment 1 comprising a frame 2 having a place 3 for a medical apparatus 4 such as an anesthesia machine or an ultrasound machine or a ventilator or an incubator or a mobile imaging device. The place 3 can be any place of the frame 2, e.g. on or above an upper level 5 of the frame 2 such as table or inside the frame 2, where the medical apparatus 4 can be installed. The movable equipment 1 further comprises a moving member 6 for allowing its movement from one location to another location in a medical environment for instance in an operating room or a critical care unit at a hospital. The moving member 6 may be one or more wheels coupled to the frame 2 of the movable equipment 1 in which case the movable equipment 1 stands on a floor or a table on its at least one wheel. Also the moving member 6 can be an arm as shown in Figure 2 fixed to an element 17 such as a wall or a ceiling or to a patient bed or some other suitable equipment making possible to move the movable equipment 1 from one location to another location. The moving member 6, which is the arm, can be equipped with one or more joints 20 to allow the movement.

The medical apparatus 4 of Figure 1 may include a vaporizer 7 and/or a ventilator 8. Pumps for intravenous drugs have not been shown in Figure 1, but they can be included in case the intravenous drugs are used. The vaporizer 7 is for producing an anesthetic agent vapor for a patient's breathing. The ventilator 8 is for controlling the patient's breathing function. The medical apparatus 4 may also comprise a user interface 9 for feeding a desired information and a display 10 for showing any information for controlling the medical apparatus 4 and the patient (not shown). The user interface 9 and the display 10 are connected to a computer 14 of the medical apparatus 4.

The movable equipment 1 comprises at least one light source 11, which is powered from a controllable AC/DC power supply 12 of the medical apparatus 4 that has a battery backup to enable a lightning during the transportation. The power can also be obtained from another power supply (not shown) dedicated only to the light source 11 with the battery backup or similar. The light source 11 can be a light emitting diode, a fluorescent lamp, a halogen lamp, a glow bulb, a gas discharge lamp etc. The light source 11 is adapted to illuminate at least a part of a space 13 adjacent to the frame 2 of the movable equipment 1. In some cases it is better to illuminate the whole space adjacent to the frame 2 of the movable equipment 1. Most important thing is to illuminate a space 13 such as a floor close to the frame 2, which may be both the floor around the frame 2 and below the frame 2 or only one of them. A suitable place for the light source 11 can be anywhere in the movable equipment 1 and depends naturally on the space 13 which should be illuminated. If the space 13 is the floor, then the good place for the light source 11 is on a lower part of the movable equipment 1, which may be a bottom 18 or a sidewall 19, or the light source 11 may also be installed on the upper level 5 or below the upper level 5 of the frame 2 as shown in Figure 2, where the light source 11 is under an edge of the upper level 5, but then the light source 11 or its light should be turned towards the floor.

The at least one light source 11 may improve a patient safety and may reduce occupational accidents. Also it improves a usability of the movable equipment I and an efficiency of the whole process encountered in the operating room and enables a safe transportation. It is important to light up the floor, cables and tubes in the health care environment and especially in the operating room. That way the user can avoid damaging cables and tubes. Same light helps safe transportation because the user can see doorsills and other obstacles under or around the transport equipment 1.

The power supply 12 communicating with the computer 14 has advantageously an ability to change an intensity and/or a color and/or frequency of the light or only one of them. The movable equipment I may be equipped with a light detector 15 to detect a brightness of an ambient light and the computer 14 can use this information to adjust automatically the intensity of the light source 11. If the ambient light is weak then the intensity of the light source 11 is increased to better illuminate the space 13. On the other hand if the ambient light is strong then the intensity of the light source 11 can be decreased. The intensity and the color of the light can also be used to reveal a condition of the medical apparatus 4 or the patient. The color can be changed by changing a wavelength of the light or by using mixed wavelengths. Changing the frequency of the light can indicate a severity of the alarm, a rapidly flashing light identifies critical alarms more easy. A slowly flashing light can mean not so severe alarm.

The light information allows the user to see from far away what is the current condition of the medical apparatus 4 or the patient. This can be considered as an initial alarm when the user can see changing conditions beforehand. The light source 11 can change its color to red if the alarm is likely to occur soon according to the data acquired from the computer 14. For example green may mean the battery status of the medical apparatus 4 is good and red may mean that the battery is empty or almost empty. A loss of the mains power can be indicated from the changing color of the light. Passing/not passing automated tests after machine power-on (green light) or failure in machine (red light) are good examples also. The color can also indicate the state of an anesthesia or a surgery operation, the user could see from red light if the patient is asleep or from green light if he is awake. Also an identification of the specific medical apparatus 4 among other apparatuses is easy because of the light.

Further the movable equipment 1 may comprise a light source 16 for an ultraviolet emission for disinfecting the floor preventing bacterial and virus contaminations and which light source 16 is also connected to the power supply 12. By using ultraviolet frequency a need of cleaning can be reduced.

The differences between embodiments of Figure 1 and Figure 2 are the location of the light source 11 and the nature of the moving member 6 as discussed above. All other features are same as explained above.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A movable equipment (1) for a medical environment, comprising:
a frame (2) having a place (3) for a medical apparatus (4);
a moving member (6) for moving said frame (2); and
at least one light source (11) for illuminating at least a part of a space (13) adjacent to said frame (2),
**characterized in that** said at least one light source (11) is on a lower part of the movable equipment and is configured to illuminate a floor around said frame (2) or a floor below said frame (2).

2. A movable equipment (1) according to claim 1, **characterized in that** at least one light source (11) is attached to a bottom (18), a sidewall (19), or an upper level (5) of the frame (2).

3. A movable equipment according to claim 1, **characterized in that** said medical apparatus (4) is one of an ultrasound apparatus, a ventilator, an incubator or a mobile imaging device.

4. A movable equipment (1) according to claim 1, **characterized in that** said medical apparatus (4) is an anesthesia machine having a ventilator (8) and vaporizer (7).

5. A movable equipment (1) according to claim 1, **characterized in that** said at least one light source (11) is adapted to change a frequency of a light.

6. A movable equipment (1) according to claim 1, **characterized in that** said at least one light source (11) is adapted to emit various intensities adjustable according to ambient conditions.

7. A movable equipment (1) according to claim 1, **characterized in that** said at least one light source (11) is adapted to create various colors, each of colors is indicative of a specific condition of a patient or a medical apparatus.

8. A movable equipment (1) according to claim 1, **characterized in that** said movable equipment further comprises a light source (16) for ultraviolet emission.

## Patentansprüche

1. Bewegliche Ausrüstung (1) für eine medizinische Umgebung, umfassend:
einen Rahmen (2) mit einem Platz (3) für eine medizinische Vorrichtung (4);
ein bewegliches Teil (6) zum Bewegen des Rahmens (2); und
zumindest eine Lichtquelle (11) zum Beleuchten zumindest eines Teils eines Raums (13) angrenzend an den Rahmen (2),
**dadurch gekennzeichnet, dass** sich die zumindest eine Lichtquelle (11) auf einem unteren Teil der beweglichen Ausrüstung befindet und ausgebildet ist, um einen Boden um den Rahmen (2) oder einen Boden unter dem Rahmen (2) zu beleuchten.

2. Bewegliche Ausrüstung (1) nach Anspruch 1, **dadurch gekennzeichnet, dass** zumindest eine Lichtquelle (11) an einem Boden (18), einer Seitenwand (19) oder einer oberen Etage (5) des Rahmens (2) angebracht ist.

3. Bewegliche Ausrüstung nach Anspruch 1, **dadurch gekennzeichnet, dass** die medizinische Vorrichtung (4) ein Ultraschallgerät oder ein Beatmungsgerät oder ein Inkubator oder eine mobile Bildgebungsvorrichtung ist.

4. Bewegliche Ausrüstung (1) nach Anspruch 1, **dadurch gekennzeichnet, dass** die medizinische Vorrichtung (4) ein Anästhesiegerät ist, das einen Beatmer (8) und einen Verdampfer (7) aufweist.

5. Bewegliche Ausrüstung (1) nach Anspruch 1, **dadurch gekennzeichnet, dass** die zumindest eine Lichtquelle (11) angepasst ist, um eine Frequenz eines Lichts zu verändern.

6. Bewegliche Ausrüstung (1) nach Anspruch 1, **dadurch gekennzeichnet, dass** die zumindest eine Lichtquelle (11) angepasst ist, um verschiedene Intensitäten auszusenden, die in Übereinstimmung mit Umgebungsbedingungen einstellbar sind.

7. Bewegliche Ausrüstung (1) nach Anspruch 1, **dadurch gekennzeichnet, dass** die zumindest eine Lichtquelle (11) angepasst ist, um verschiedene Farben zu erzeugen, wobei jede der Farben ein Indikator für einen spezifischen Zustand eines Patienten oder einer medizinischen Vorrichtung ist.

8. Bewegliche Ausrüstung (1) nach Anspruch 1, **dadurch gekennzeichnet, dass** die bewegliche Ausrüstung weiterhin eine Lichtquelle (16) für ultraviolette Emission umfasst.

## Revendications

1. Equipement mobile (1) destiné à un environnement médical, comprenant :
une structure (2) comportant un emplacement (3) pour un appareil médical (4);
un élément de déplacement (6) destiné à déplacer ladite structure (2) ; et
au moins une source de lumière (11) destinée à éclairer au moins une partie d'un espace (13) adjacent à ladite structure (2),
**caractérisé en ce que** ladite au moins une source de lumière (11) est sur une partie inférieure de l'équipement mobile et est configurée de manière à éclairer un plancher autour de ladite structure (2) ou un plancher au-dessous de ladite structure (2).

2. Equipement mobile (1) selon la revendication 1, **caractérisé en ce qu'**au moins une source de lumière (11) est fixée sur un fond (18), une paroi latérale (19), ou un niveau supérieur (5) de la structure (2).

3. Equipement mobile selon la revendication 1, **caractérisé en ce que** ledit appareil médical (4) est l'un d'un appareil à ultrasons, d'un ventilateur, d'un incubateur ou d'un dispositif d'imagerie mobile.

4. Equipement mobile (1) selon la revendication 1, **caractérisé en ce que** ledit appareil médical (4) est une machine d'anesthésie comportant un ventilateur (8) et un vaporisateur (7).

5. Equipement mobile (1) selon la revendication 1, **caractérisé en ce que** ladite au moins une source de lumière (11) est adaptée afin de modifier une fréquence d'une lumière.

6. Equipement mobile (1) selon la revendication 1, **caractérisé en ce que** ladite au moins une source de lumière (11) est adaptée afin d'émettre différentes intensités réglables en fonction des conditions ambiantes.

7. Equipement mobile (1) selon la revendication 1, **caractérisé en ce que** ladite au moins une source de lumière (11) est adaptée afin de créer différentes couleurs, chacune des couleurs est représentative d'un état spécifique d'un patient ou d'un appareil médical.

8. Equipement mobile (1) selon la revendication 1, **caractérisé en ce que** ledit équipement mobile comprend, en outre, une source de lumière (16) assurant une émission en ultraviolet.
